**Europäisches Patentamt**

**European Patent Office**

**Office européen des brevets**

(19)

(11) Veröffentlichungsnummer: **0 005 432**
**B1**

(12) # EUROPÄISCHE PATENTSCHRIFT

(45) Veröffentlichungstag der Patentschrift:
12.05.82

(21) Anmeldenummer: **79100380.9**

(22) Anmeldetag: **09.02.79**

(51) Int. Cl.³: **G 01 S 7/62,** G 01 S 15/02,
H 04 N 7/00, A 61 B 10/00

(54) **Nach dem Impuls-Echoverfahren arbeitendes Ultraschall-Bildgerät.**

(30) Priorität: **11.05.78 DE 2820660**

(43) Veröffentlichungstag der Anmeldung:
**28.11.79 Patentblatt 79/24**

(45) Bekanntmachung des Hinweises auf die Patenterteilung:
**12.05.82 Patentblatt 82/19**

(84) Benannte Vertragsstaaten:
**CH FR GB NL**

(56) Entgegenhaltungen:
**DE-A-2 629 895**
**FR-A-2 310 608**
**NL-A-7 708 143**
**US-A-3 947 826**
**US-A-4 121 250**
**JEE (JOURNAL OF ELECTRONIC ENGINEER-**
**ING),**
**no. 132, Dezember 1977, TOKYO (JP) ITO und**
**Andere:**
**»A real time ultrasonic diagnostic system**
**for dynamic and still images:**
**Wireless Echovision«, Seiten 20—26**

(73) Patentinhaber: **SIEMENS AKTIENGESELLSCHAFT**
**Berlin und München, Postfach 22 02 61,**
**D-8000 München 22 (DE)**

(72) Erfinder: **Buchner, Klaus, Ing.grad., Schleifweg 7 a,**
**D-8521 Uttenreuth (DE)**
Erfinder: **Haerten, Rainer, Dr. Dipl.-Phys., Im**
**Heuschlag 25, D-8520 Erlangen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Nach dem Impuls-Echoverfahren arbeitendes Ultraschall-Bildgerät

Die Erfindung bezieht sich auf ein nach dem Impuls-Echoverfahren arbeitendes Ultraschall-Bildgerät, insbesondere für die medizinische Diagnostik, mit Ultraschall-Applikator für die zeilenweise Ultraschallabtastung eines Untersuchungsobjektes und Bildanzeigevorrichtung mit Zeilengenerator für die Abbildung der Echoimpulse als Zeile sowie Bildgenerator für die Verschiebung der Zeile in Abhängigkeit von der Verschiebung des Ultraschallstrahles im Objekt, wobei ausgehend von einer vorgebbaren Zeilenkippzeit einer Bildröhre als Bildanzeigevorrichtung, z. B. der Norm-Zeilenkippzeit einer Fernsehbildröhre, die Periodenzeit der Ultraschall-Sende/Empfangszyklen des Ultraschall-Applikators auf einen solchen Wert eingestellt ist, der einem ganzzahligen Vielfachen der Zeilenkippzeit der Bildröhre entspricht und wobei im Takt dieser Sende/Empfangszyklen anfallende Echosignale für jeden Zyklus bis zum nächstfolgenden in Wechselspeichern gespeichert und erst während dieses nächstfolgenden Zyklus zur Bildröhre ausgelesen werden, wobei das Auslesen ein- oder mehrfach erfolgt in solchen Auslesezeiten, die der Zeilenkippzeit der Bildröhre entsprechen, und wobei den Wechselspeichern ein weiterer Speicher zugeordnet ist.

Ein solches Bildgerät ist z. B. durch die DE-OS 2 629 895 bekannt. Es ermöglicht speziell eine Video-Norm-Umsetzung mit lediglich maximal zwei Speichern zur Zwischenspeicherung der Echosignale jeweils zweier aufeinanderfolgender Abtastzeilen. Die Umsetzung des Ultraschallbildes erfolgt dabei im Direktverfahren.

Interessant ist in der Praxis nun nicht nur allein die Direktdarstellung des unmittelbar zu diesem Zeitpunkt abgetasteten Bildes; von Bedeutung ist auch die Wiedergabe gespeicherter Bilder. Hierdurch erhält man ein stehendes Bild, das die Vermessung des Ultraschallbildes am Bildschirm erleichtert. Auch sonst wird durch stehende Bilder die diagnostische Aussage, beispielsweise bei späterer Reproduktion, erleichtert.

Zur Darstellung digital gespeicherter Bilder, insbesondere in Fernsehnorm, waren bisher technisch sehr aufwendige Gesamtbildspeicher (sog. Orthogonalspeicher) nötig, mit deren Hilfe also nur ein bereits umgesetztes komplettes Bild gespeichert und wieder abgerufen werden konnte. Ultraschall-Bildgeräte mit einem derartigen Speichersystem sind beispielsweise aus der DE-OS 2 619 684 (= FR-A 2 310 608), NL-A 7 708 143 (= US-PS 4 121 250) oder auch aus der Zeitschrift JEE (Journal of Electronic Engineering), No. 132, Dez. 1977, Seiten 20 bis 26 vorbekannt. Die US-PS 3 947 826 beinhaltet hingegen einen Bildkonverter zur Umwandlung langsam anfallender Abtastbilder in ein schnelles TV-Bild einer Fernsehbildröhre auf der Basis rein von Wechselspeichern, denen kein weiterer Speicher zugeordnet ist.

Aufgabe vorliegender Erfindung ist es, ein Bildgerät für Umwandlung von Ultraschallbildern in eine vorgebbare Bildart, z. B. in Fernsehnorm, zu schaffen, das neben Direktwiedergabe auch die Wiedergabe gespeicherter Ultraschallbilder bei kleinstem technischen Aufwand ermöglicht. Die Wiedergabe gespeicherter Bilder sollte vorzugsweise auch gleichzeitig mit einem aktuellen Bild erfolgen können.

Die Aufgabe wird mit einem Ultraschall-Bildgerät der eingangs genannten Art dadurch gelöst, daß der den Wechselspeichern zugeordnete Speicher speziell ein Echozeilen-Zusatzspeicher ist, der bezüglich des Signalweges für aktuelle Echozeilen eines Ultraschall-Bildes zwischen Ultraschall-Applikator und den Wechselspeichern so angeordnet ist, daß er zum aktuellen Signalweg parallel liegt, so daß aktuelle Echozeilen eines Ultraschall-Bildes in den Echozeilen-Zusatzspeicher einspeicherbar und gespeicherte Zeilen durch Anschalten des Speicherausgangs am Signalweg vor den Wechselspeichern bei Bedarf wieder entsprechend Zeile für Zeile über die Wechselspeicher zur Bildröhre auslesbar sind.

Die Erfindung ermöglicht lediglich durch Einsatz eines technisch wenig aufwendigen Echozeilen-Zusatzspeichers anstelle eines aufwendigen und teuren Gesamtbildspeichers die Bildaufzeichnung von vorher gespeicherten Ultraschall-Echobildern in erwünschter Wiedergabeart. Die Aufzeichnung der gespeicherten Bilder kann unabhängig von der Darstellung aktueller Bilder erfolgen; ebensogut ist bei Bedarf jedoch auch die Aufzeichnung gespeicherter Bilder zusammen mit der Darstellung eines aktuellen Bildes möglich.

Weitere Vorteile und Einzelheiten der Erfindung ergeben sich aus der nachfolgenden Beschreibung eines Ausführungsbeispiels anhand der Zeichnung in Verbindung mit den Unteransprüchen.

Es zeigt

Fig. 1 ein Ultraschall-Bildgerät gemäß der Erfindung speziell zur Umsetzung in Fernsehnorm im Prinzipschaltbild,

Fig. 2 ein Impulsdiagramm zur Durchführung einer Video-Norm-Umsetzung gemäß der Erfindung mit dem Prinzipschaltbild nach Fig. 1.

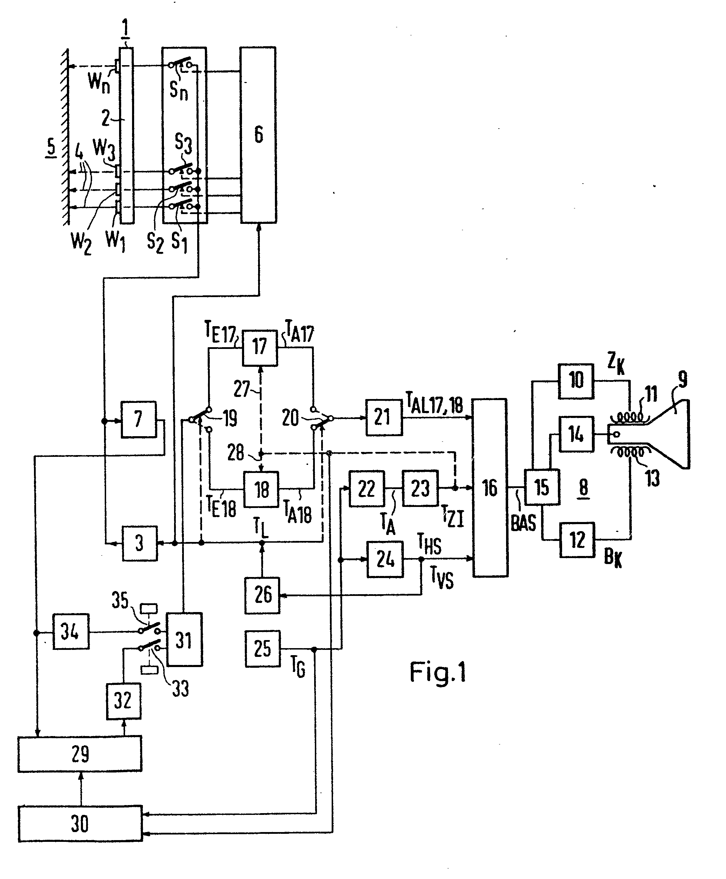

In der Fig. 1 ist mit 1 der Ultraschall-Applikator bezeichnet, der im vorliegenden Fall als Ultraschall-Array ausgebildet ist. Der Applikator 1 besteht demnach aus einer Vielzahl von Ultraschallwandlern $W_1$ bis $W_n$ (piezoelektrische Kristallplättchen), die in Reihe nebeneinander an einem Trägerteil 2 aus Ultraschallwellen gut dämpfendem Material gehaltert sind. Die einzelnen Wandlerelemente $W_1$ bis $W_n$ sind wahlweise einzeln oder gruppenweise durch Hochfrequenzimpulse eines Hochfrequenzimpulsgenerators 3 erregbar in dem Sinne, daß sie Ultraschallimpulse in Richtung der Pfeile 4 in ein Untersuchungsobjekt 5, beispielsweise menschlichen Körper, einstrahlen. Die Ansteuerung der einzelnen Wandlerelemente $W_1$ bis $W_n$ in Einzel- oder Gruppenformation erfolgt mittels einer Ansteuervorrichtung, die in der üblichen Weise Ansteuerschieberegister 6 sowie Ansteuerschalter $S_1$ bis $S_n$ zur Anschaltung zu erregender Wandlerelemente an den Hochfrequenzimpulsgenerator 3 im Sendebetrieb bzw. an einen Echoimpulsempfangsverstärker 7 im Empfangsbetrieb umfaßt. Die Ansteuervorrichtung arbeitet dabei mit dem Schieberegister 6 in der Weise, daß durch entsprechend angesteuerte Schalter $S_1$ bis $S_n$ die einzelnen Wandlerelemente $W_1$ bzw. $W_n$ über die Wandlerreihe fortlaufend nacheinander einzeln — oder gruppenweise — auf Senden bzw. Empfang schaltbar sind. Auf diese Weise ergibt sich ein zeilenweises Fortschreiten des Ultraschallsende/Empfangsstrahles über die Wandlerreihe hinweg und damit entsprechend zeilenweise Ultraschallabtastung des Untersuchungsobjektes 5. Zur Aufzeichnung der Echosignale jeder Ultraschall-Abtastzeile als entsprechende Bildzeile dient ein herkömmlicher Fernsehmonitor 8. Dieser Fernsehmonitor umfaßt eine Fernsehbildröhre 9, der in üblicher Weise ein Zeilenkippgenerator 10 für eine Horizontalablenkspule 11 sowie ein Bildkippgenerator 12 für die Vertikalablenkspule 13 der Fernsehbildröhre 9 zugeordnet ist. Der Monitor 8 umfaßt ferner auch noch einen Helligkeitsmodulator 14 zur Helligkeitsmodulation der Bildzeilen des Zeilenrasters im Takt der anfallenden Echoimpulse sowie eine Impulstrennstufe 15 für die unterschiedlichen Informationsinhalte des dem Monitor 8 zugeleiteten BAS-Signals.

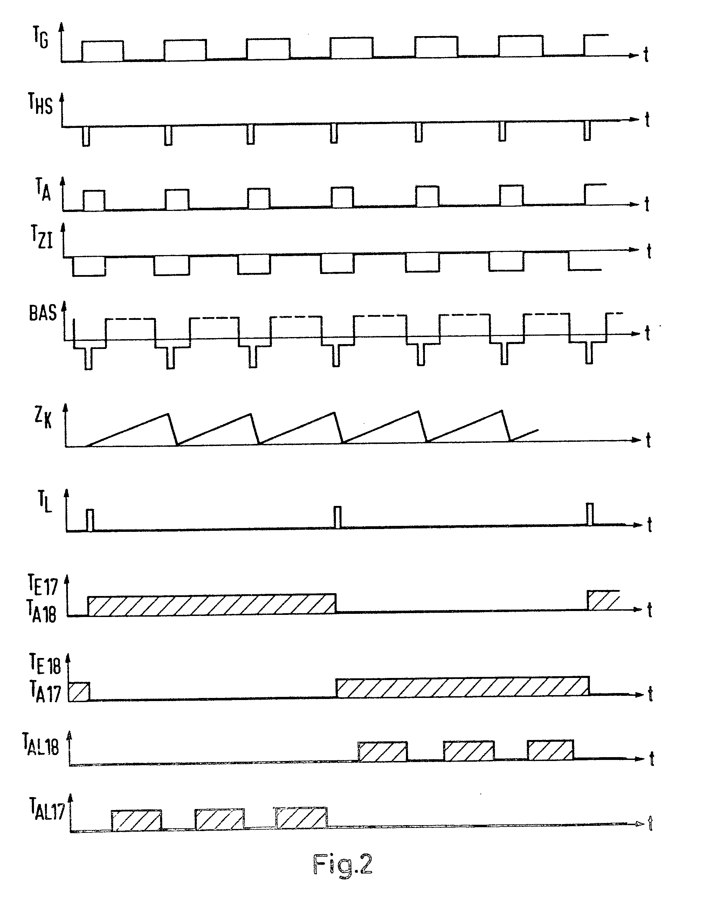

Das BAS-Signal fällt dabei am Ausgang einer Mischstufe 16 an, die eingangsseitig von den Echosignalinformationen, den Horizontal-(bzw. Vertikal-)Austastimpulsen sowie den Zeilen-(bzw. Bild-)Synchronimpulsen gespeist ist. Speziell im Direktabtastbetrieb werden die Echosignalinformationen vom Ausgang des Echosignalempfangsverstärkers 7 über eine später noch näher zu erläuternde Serienschaltung aus einem Pufferspeicher 34 mit Schalter 35 und Mischstufe 31 zu Pufferspeichern 17 bzw. 18, die mittels oszillierender Schalter 19 und 20 im Gegentaktbetrieb arbeiten, geführt und von dort über einen weiteren Verstärker 21 für die Echosignale schließlich auf die BAS-Mischstufe 16 gegeben. Zur Erzeugung der Horizontal- (bzw. Vertikal-)Austastimpulse bzw. der Zeilen- (bzw. Bild-)Synchronisierimpulse dienen ein Austastimpulserzeuger 22, 23 bzw. ein Synchronimpulserzeuger 24. Der Austastimpulserzeuger umfaßt zwei monostabile Kippstufen 22 und 23, die im Takt von Taktimpulsen $T_G$ eines Grundtaktgebers 25 zeitlich nacheinander angestoßen werden. Aus dem Grundtakt des Grundtaktgebers 25, der mit der normierten Zeilenkippfrequenz der Fernsehbildröhre 9 (von z. B. 15,625 kHz) schwingt, ergeben sich somit gemäß Fig. 2 die beiden Impulsfolgen $T_A$ bzw. $T_{ZI}$. Der Grundtaktgeber 25 taktet ferner auch den Synchronimpulserzeuger 24, der ebenfalls als monostabile Kippstufe ausgebildet ist, welche kurzzeitige Impulse gemäß dem Taktverlauf $T_{HS}$ abgibt. In Überlagerung der jeweiligen Austastimpulse mit den Synchronimpulsen und den Echoinformationen ergibt sich dann das BAS-Signal in dem zeitlichen Verlauf gemäß Fig. 2. Nach Abtrennung in der Impulstrennstufe 15 des Fernsehmonitors 8 wird dann durch die Horizontalsynchronimpulse jeweils die Zeilenkippspannung $Z_K$ ausgelöst. Am Ende eines jeden Bildaufbaues wird hingegen der Bildsynchronimpuls in Verbindung mit einem Bildaustastimpuls erzeugt, der die Zurückführung des Elektronenstrahles der Fernsehbildröhre 9 in die Ausgangslage und Wiederauslösung eines weiteren Bildkipps in Verbindung mit weiteren Zeilenrasterkipps bewirkt. Zur Festlegung der Sende/Empfangszeiten der Ultraschall-Sendeempfangszyklen dient hingegen ein Zähler 26, in den die Synchronimpulse des Synchronimpulserzeugers 24 eingezählt werden.

Der Zähler 26 erzeugt gemäß vorliegendem Ausführungsbeispiel mit jedem vierten anfallenden Synchronimpuls einen Ausgangsimpuls $T_L$, der als Leittaktimpuls einerseits Weitertaktung in den Registerpositionen des Schieberegisters 6 und damit rhythmische Weiterschaltung der Schalter $S_1$ bis $S_n$ bewirkt. Andererseits wird auch gleichzeitig der Hochfrequenzimpulssender jeweils mit Auftreten eines Leittaktimpulses $T_L$ im Sinne der Abgabe eines Hochfrequenzimpulses an das durch einen Schalter $S_1$ bis $S_n$ jeweils angeschaltete Wandlerelement $W_1$ bis $W_n$ aktiviert. Es ergibt sich somit zeilenweise Ultraschallabtastung des Untersuchungsobjektes 5 im Takt der Leittaktimpulse $T_L$. Des weiteren erfolgt über die Leittaktimpulse $T_L$ auch noch wechselweises Umschalten der Schalter 19 bzw. 20. In der Fig. 2 sind dabei die Umschaltzeiten an den Speichereingängen bzw. die Umschaltzeiten an den Speicherausgängen jeweils mit $T_{E17}$ bzw. $T_{E18}$ und $T_{A17}$ bzw. $T_{A18}$ bezeichnet. Die schraffiert dargestellten Impulse bezeichnen jeweils die Zeitdauer der Einspeisung von Echosignalinformationen in den einen Speicher bei gleichzeitiger Auslesung der Information einer vorhergehenden Echozeile im anderen Speicher. Die tatsächliche Auslesung der Informationen aus dem jeweils ausgangsseitig angeschalteten Speicher 17 oder 18 über den Verstärker 21 zur Mischstufe 16 erfolgt hingegen im Synchrontakt der Ausgangsimpulse des Austastimpulserzeugers 22, 23 über die Auslesesteuerleitung 27 bzw. 28. Da jeweils während der Zeitdauer der Ansteuerung eines Speichers 17 bzw. 18 durch den Ausgangsschalter 20 insgesamt immer drei Austastimpulse des Austastimpulserzeugers 22, 23 in zeitlicher Aufeinanderfolge anfallen, wird entsprechend eine im jeweils angeschalteten Speicher 17 oder 18 eingespeicherte Echozeile insgesamt dreimal in zeitlicher Aufeinanderfolge ausgelesen. Am Bildschirm der Fernsehbildröhre 9 ergibt sich somit ein Echobild mit hoher Zeilendichte und relativ hoher Bildfrequenz, wobei jedoch jede Zeile dreifach geschrieben ist.

Wie beschrieben erfolgt der Betrieb bisher nur im Direktabtastverfahren, d. h. es werden solche Echozeilen, die im Abtastbetrieb direkt am Untersuchungsobjekt anfallen, entsprechend direkt in Videonorm umgesetzt und am Fernsehmonitor als Direktbild dargestellt.

Gemäß vorliegender Erfindung besteht nun jedoch auch die Möglichkeit der Abspielung und Darstellung gespeicherter Bilder; als tatsächliches Fernsehbild kann dann entweder allein das gespeicherte Bild dienen oder es können in Abwandlung auch das gespeicherte Bild (oder mehrere gespeicherte Bilder) zusammen mit einem jetzt wiederum im Direktabtastbetrieb anfallenden Direktbild in Überlagerung das Fernsehbild darstellen.

Zur Durchführung derartiger Verfahren ist demnach im Prinzipschaltbild der Fig. 1 am Ausgang des Echoimpuls-Empfangsverstärkers 7 ein Zusatzspeicher 29 (z. B. RAM-Speicher) für die anfallenden Echozeilen eines Echobildes angeschaltet. Die Steuerung des Zusatzspeichers im Sinne des Einlesens bzw. Wiederauslesens von Zeilen oder ganzen Bildern erfolgt durch eine Steuerschaltung 30 in Abhängigkeit von den Grundtaktimpulsen $T_G$ des Grundtaktgebers 25 einerseits und den Steuerimpulsen der Ein- bzw. Ausleseleitung 27, 28 für die Pufferspeicher 17 bzw. 18 andererseits. Die Auslesung gespeicherter Zeilen aus dem Zusatzspeicher 29 zu den Wechselpufferspeichern 17 bzw. 18 erfolgt über eine Mischstufe 31 bei Zwischenschaltung eines Auslesepufferspeichers 32 mit Verbindungsschalter 33. Der Mischstufe 31 wird — wie eingangs bereits beschrieben — an einem zweiten Eingang wiederum über einen Pufferspeicher 34 mit Schalter 35 auch das Ausgangssignal des Echoimpuls-Empfangsverstärkers 7 bei Zeilen- bzw. Bildübertragung im Direktbereich zugeleitet.

Die Funktionsweise ergibt sich damit wie folgt:

Während bei geschlossenem Schalter 35 vom Echoimpuls-Empfangsverstärker 7 über den Pufferspeicher 34 und die Mischstufe 31 ein Fernsehbild im Direktverfahren erzeugt wird, werden zur gleichen Zeit im Takt von Steuersignalen der Steuerschaltung 30 die aktuellen Echosignale zeilenweise nacheinander in den Zusatzspeicher 29 eingeschrieben. Die Einschreibfrequenz in den Zusatzspeicher entspricht exakt der Einschreibfrequenz entsprechender Zeilen in die Pufferspeicher 17 bzw. 18. Die Kapazität des Zusatzspeichers 29 ist auf die Anzahl der Echozeilen wenigstens eines Ultraschallbildes, multipliziert mit den Abtastpunkten jeder Zeile und der bit-Tiefe, beschränkt. Je nach Stellung der Schalter 33 und 35 erhält dann der Mischer 31 im Auslesebetrieb wahlweise entweder Echosignale nur vom Echoimpuls-Empfangsverstärker 7 über den Pufferspeicher 34 oder nur vom Zusatzspeicher 29 über den Auslesepufferspeicher 32 oder von beiden gleichzeitig. Die Fernsehnormwandlung erfolgt bei sämtlichen möglichen Betriebsweisen nach wie vor über die Wechselpufferspeicher 17, 18 in der Mischstufe 16.

Ist die Auslesefrequenz des Zusatzspeichers 29 gleich seiner Einschreibefrequenz, so entspricht das gespeicherte Bild im Maßstab jenem, das im Direktabtastbetrieb gewonnen wurde. Bei Abweichungen der Auslesefrequenz zu höheren oder tieferen Werten ergibt sich eine entsprechende Verkleinerung oder Vergößerung des Maßstabes. Damit kann z. B. dem Direktbild (Echtzeitbild) ein gespeichertes verkleinertes Gesamtbild, Teilbild od. dgl. bzw. ein solches mit Ausschnittsvergrößerung zugemischt werden. In diagnostischer Hinsicht können somit anhand des Direktbildes und des überlagerten gespeicherten, z. B. auch maßstabsgeänderten, Teilbildes beliebige Vergleichsmessungen durchgeführt werden.

Der Parallelbetrieb von Zusatzspeicher 29 und Zeilenwechselpuffer-Speicher 17 und 18 erlaubt eine einfache zeilenweise Organisation des Zusatzspeichers im Schreib- und Lese-Modus und damit eine einfache Adressierung der nacheinanderfolgenden Bildpunkte einer jeden Zeile.

Das beschriebene Ausführungsbeispiel arbeitet im einfachen Vollbildverfahren. Selbstverständlich kann jedoch auch ein Zeilensprungverfahren angewendet werden in dem Sinne, daß bei geradzahligem Vielfachen der Horizontalsynchronimpulse Ultraschallabtastung im Untersuchungsobjekt und Zeilenbildaufzeichnung mit der Fernsehbildröhre in z. B. zwei aufeinanderfolgenden Halbbildern geschieht, wobei die Mehrfachschreibung einer Echozeile die Hälfte des geradzahligen Vielfachen des Horizontalsynchrons beträgt.

**Patentansprüche**

1. Nach dem Impuls-Echoverfahren arbeitendes Ultraschall-Bildgerät, insbesondere für die medizinische Diagnostik, mit Ultraschall-Applikator (1) für die zeilenweise Ultraschallabtastung eines Untersuchungsobjektes (5) und Bildanzeigevorrichtung (9) mit Zeilengenerator (10) für die Abbildung der Echoimpulse als Zeile sowie Bildgenerator (12) für die Verschiebung der Zeile in Abhängigkeit von der Verschiebung des Ultraschallstrahles im Objekt, wobei ausgehend von einer vorgebbaren Zeilenkippzeit einer Bildröhre als Bildanzeigevorrichtung, z. B. Norm-Zeilenkippzeit einer Fernsehbildröhre, die Periodenzeit der Ultraschall-Sende/Empfangszyklen des Ultraschall-Applikators auf einen solchen Wert eingestellt ist, der einem ganzzahligen Vielfachen der Zeilenkippzeit der Bildröhre entspricht, und wobei im Takt dieser Sende/Empfangszyklen anfallende Echosignale für jeden Zyklus bis zum nächstfolgenden in Wechselspeichern (17, 18) gespeichert und erst während dieses nächstfolgenden Zyklus zur Bildröhre ausgelesen werden, wobei das Auslesen ein- oder mehrfach erfolgt in solchen Auslesezeiten, die der Zeilenkippzeit der Bildröhre entsprechen, und wobei den Wechselspeichern ein weiterer Speicher zugeordnet ist, dadurch gekennzeichnet, daß der den Wechselspeichern (17, 18) zugeordnete Speicher speziell ein Echozeilen-Zusatzspeicher (29) ist, der bezüglich des Signalweges für aktuelle Echozeilen eines Ultraschall-Bildes zwischen Ultraschall-Applikator (1) und den Wechselspeichern (17, 18) so angeordnet ist, daß er zum aktuellen Signalweg parallel liegt, so daß aktuelle Echozeilen eines Ultraschall-Bildes in den Echozeilen-Zusatzspeicher (29) einspeicherbar und gespeicherte Zeilen durch Anschalten des Speicherausganges am Signalweg vor den Wechselspeichern (17, 18) bei Bedarf wieder entsprechend Zeile für Zeile über die Wechselspeicher zur Bildröhre (9) auslesbar sind.

2. Ultraschall-Bildgerät nach Anspruch 1, gekennzeichnet durch eine Steuerschaltung (30) zur Steuerung des Zusatzspeichers (29) in dem Sinne, daß aktuell anfallende Zeilen eines Ultraschall-Echobildes gleichzeitig mit ihrer Weiterleitung über die Wechselspeicher (17, 18) zur Bildröhre (9) zeilenweise in den Zusatzspeicher (29) eingeschrieben werden.

3. Ultraschall-Bildgerät nach Anspruch 1 oder 2, gekennzeichnet durch eine Steuereinrichtung (30) zur Steuerung des Zusatzspeichers (29) in dem Sinne, daß im Zusatzspeicher (29) gespeicherte Zeilen wahlweise ohne oder zusammen mit weiteren aktuellen Zeilen einer in diesem Augenblick erfolgenden Direktabtastung über die Wechselspeicher (17, 18) zur Aufzeichnung auf die Bildröhre (9) ausgelesen werden.

4. Ultraschall-Bildgerät nach einem der Ansprüche 1 bis 3, dadurch gekennzeichnet, daß durch Vorgabe ausgewählter Verhältnisse zwischen Einlese- und Wiederauslesefrequenz des Zusatzspeichers (29) im Vergleich mit aktuellen Bildern maßstabsgeänderte Speicherbilder erhalten werden.

5. Ultraschall-Bildgerät nach einem der Ansprüche 1 bis 4, dadurch gekennzeichnet, daß die Steuerung des Zusatzspeichers (29) zur Vorgabe der Ein- bzw. Auslesezeiten in Abhängigkeit von einer Steuereinrichtung (30) erfolgt, die ihrerseits vom Grundtakt ($T_G$) eines Grundtaktgebers (25) und den Schreibimpulsen der Schreibsteuerleitungen (27, 28) für die Wechselspeicher (17, 18) gesteuert ist.

6. Ultraschall-Bildgerät nach einem der Ansprüche 1 bis 5, dadurch gekennzeichnet, daß die Zuleitung von aktuellen Zeilen des Ultraschall-Applikators (1) bzw. gespeicherten Zeilen des Zusatzspeichers (29) zu den Wechselspeichern (17, 18) über eine Mischstufe (31) erfolgt, der Eingangsschalter (33, 35) zugeordnet sind, die eine Verbindung der Mischstufe (31) wahlweise

a)    nur mit den aktuellen Zeilen am Ausgang des Ultraschall-Applikators (1) oder

b)    nur mit den Ausgangszeilen aus dem Inhalt des Zusatzspeichers (29) oder

c)    mit beiden gleichzeitig

ermöglichen.

7. Ultraschall-Bildgerät nach Anspruch 6, dadurch gekennzeichnet, daß in den Verbindungen der Mischstufe (31) zum Ultraschall-Applikator (1) einerseits und zum Ausleseausgang des Zusatzspeichers (29) andererseits je ein Pufferspeicher (32 bzw. 34) eingeschaltet ist.

8. Ultraschall-Bildgerät nach Anspruch 6 oder 7, dadurch gekennzeichnet, daß die Steuerung der Auswahlschalter (33, 35) vor der Mischstufe (31) von Hand, z. B. als Tastenschalter, oder selbsttätig, z. B. in Abhängigkeit von Steuersignalen der Steuerschaltung (30) für den Zusatzspeicher (29), erfolgt.

## Claims

1. Ultrasonic display apparatus using pulse echo techniques, in particular for medical diagnostics, comprising an ultrasonic applicator (1) for the ultrasonic linear scanning of a test object (5) and a picture display device (9) which has a line generator (10) for the representation of the echo pulses as a line and a picture generator (12) for the displacement of the line in dependence upon the displacement of the ultrasonic ray in the object, wherein commencing from a pre-determinable line-sweep time of a display tube as picture displaying device, e. g. the standard line-sweep time of the television tube, the period of the ultrasonic transmitting/receiving cycles of the ultrasonic applicator are adjusted to a value which corresponds to an integral multiple of the line-sweep time of the picture tube, and wherein for each cycle the echo signals which occur in the timing of these transmitting/receiving cycles are stored in change stores (17, 18) until the next following cycle and only read out to the display tube during this next following cycle, wherein the reading occurs once or repeatedly in reading times which correspond to the line-sweep time of the display tube, and wherein the change stores are assigned a further store, characterised in that the store assigned to the change stores (17, 18) is specifically an echo line additional store (29) which in respect of the signal path for actual echo lines of an ultrasonic picture, is arranged between the ultrasonic applicator (1) and the change stores (17, 18) in such manner that it is parallel to the actual signal path, so that actual echo lines of an ultrasonic picture can be stored in the echo line additional stores (29) and if required, stored lines can be re-read to the display tube (9) line by line via the change stores by connecting the storage output on the signal path before the change stores (17, 18).

2. Ultrasonic television apparatus as claimed in claim 1, characterised by a control circuit (30) for controlling the additional store (29) to the effect that actually occurring lines of an ultrasonic echo picture are read into the additional store (29) line by line simultaneously with their advance to the display tube (9) via the change stores (17, 18).

3. Ultrasonic television apparatus as claimed in claim 1 or 2, characterised by a control device (30) for controlling the additional store (29) to the effect that lines which are stored in the additional store (29) are read out via the change stores for the recording onto the display tube (9) alternatively without or together with further actual lines of a direct scanning which occurs at this moment.

4. Ultrasonic television apparatus as claimed in one of the claims 1 to 3, characterised in that storage pictures which are scaled in comparison with actual pictures are received by determining selected ratios between the read-in frequency and the read-out frequency of the additional store (29).

5. Ultrasonic television apparatus as claimed in one of the claims 1 to 4, characterised in that in order to determine the read-in and read-out times the control of the additional store (29) occurs in dependence upon a control device (20) which on its part is controlled by the basic cycle (T$_G$) of a basic cycle generator (25) and by the writing pulses of the recording control lines (27, 28) for the change stores (17, 18).

6. Ultrasonic display apparatus as claimed in one of the claims 1 to 5, characterised in that the supply of actual lines of the ultrasonic applicator (1) or the stored lines of the additional store (29) as the case may be, to the change stores (17, 18) is carried out via a mixing stage (31) which is assigned input switches (33, 35) which allow a connection of the mixing stage (31) alternatively

a) only to the actual lines at the output of the ultrasonic applicator (1); or
b) only to the output lines from the content of the additional store (29); or
c) to both simultaneously.

7. Ultrasonic television apparatus as claimed in claim 6, characterised in that in the connections of the mixing stage (31) to the ultrasonic applicator (1) on the one hand and to the read-out output of the additional store (29) on the other hand, there is respectively interpolated a buffer store (32 and 34).

8. Ultrasonic television apparatus as claimed in claim 6 or 7, characterised in that the control of the selection switches (33, 35) is carried out manually, e. g. as key switch, or automatically, e. g. in dependence upon control signals of the control circuit (30) for the additional store, preceding the mixing stage (31).

## Revendications

1. Appareil pour produire des images ultrasonores opérant suivant le procédé par échos d'impulsions, plus particulièrement pour le diagnostic médical, comportant un applicateur d'ultrasons (1) pour le balayage ultrasonore et ligne par ligne de l'objet à examiner (5) et un dispositif d'affichage de l'image (9) à générateur de ligne pour la reproduction des impulsions d'échos comme ligne et à générateur d'image (12) pour le décalage de la ligne en fonction du décalage du rayon ultrasonore dans l'objet, du type dans lequel, à partir d'une durée de balayage des lignes prédéterminée d'un tube image servant de dispositif d'affichage des images, par exemple la durée de balayage des lignes normalisée d'un tube de télévision, la durée de la période des cycles émission/réception ultrasonore de l'applicateur ultrasonore est réglée à une valeur qui correspond à un multiple entier de la durée de balayage des lignes du tube-image, et dans lequel les signaux d'échos qui se présentent à la cadence de ces cycles émission/réception sont mémorisés pour chaque cycle jusqu'au suivant dans des mémoires alternées pour n'être lus pour le tube-image que pendant ledit cycle suivant, alors que la lecture a lieu une ou plusieurs fois pendant des durées de lecture qui correspondent à la durée de balayage des lignes du tube-image et qu'aux mémoires alternées est associée une mémoire supplémentaire, caractérisé par le fait que la mémoire associée aux mémoires alternées (17, 18) est particulièrement une mémoire supplémentaire des lignes d'échos (29) qui est, pour ce qui concerne la voie des signaux pour les lignes d'échos actuelles d'une image ultrasonore, disposée de telle manière entre l'applicateur ultrasonore (1) et les mémoires alternées (17, 18) qu'elle est en parallèle sur la voie des signaux actuelle, en sorte que les lignes d'échos actuelles d'une image ultrasonore sont susceptibles d'être mémorisées dans la mémoire supplémentaire des lignes d'échos (29) et que par le branchement de la sortie de la mémoire en avant des mémoires alternées (17, 18) des lignes mémorisées peuvent être envoyées par l'intermédiaire des mémoires alternées aux images (9), à nouveau ligne par ligne, si besoin est.

2. Appareil pour produire des images ultrasonores selon la revendication 1, caractérisé par un circuit de commande (30) pour la commande de la mémoire supplémentaire (29) dans le sens tel que les lignes actuelles d'une image d'échos ultrasonores qui se présentent sont inscrites ligne par ligne dans la mémoire supplémentaire (25), en même temps qu'a lieu leur transmission, par l'intermédiaire des mémoires alternées (17, 18) vers le tube-image.

3. Appareil pour produire des images ultrasonores selon la revendication 1 ou 2, caractérisé par un dispositif de commande (30) pour la commande de la mémoire supplémentaire (29) dans un sens tel que les lignes mémorisées dans la mémoire supplémentaire (29) sont lues sans ou avec d'autres lignes actuelle d'un balayage direct réalisé à cet instant ladite lecture s'opérant par l'intermédiaire des mémoires alternées (17, 18) pour l'écriture sur le tube-image (9).

4. Appareil pour produire des images ultrasonores selon l'une des revendications 1 à 3, caractérisé par le fait qu'en donnant à l'avance des rapports choisis entre la fréquence d'écriture et la fréquence de lecture de la mémoire supplémentaire (29), on obtient des images mémorisées à échelle modifiée par rapport aux images actuelles.

5. Appareil pour produire des images ultrasonores selon l'une des revendications 1 à 4, caractérisé par le fait que la commande de la mémoire supplémentaire (29) pour donner à l'avance des durées d'écriture et de lecture est opérée en fonction d'un dispositif de commande (30) qui est, à son tour, commandé par la cadence de base ($T_G$) d'un générateur de cadence de base (25) et par les impulsions d'écriture des lignes de commande d'écriture (27, 28) pour les mémoires alternées (17, 18).

6. Appareil pour produire des images ultrasonores selon l'une des revendications 1 à 5, caractérisé par le fait que la transmission de lignes actuelles de l'applicateur ultrasonore (1) ou de lignes mémorisées de la mémoire supplémentaire (29) aux mémoires alternées (17, 18) est opérée par l'intermédiaire d'un étage mélangeur (31) auquel sont associés des commutateurs d'entrée (33, 35) qui permettent la liaison avec l'étage mélangeur (31) au choix

a) uniquement avec les lignes actuelles à la sortie de l'applicateur ultrasonore (1) ou
b) uniquement avec les lignes de sortie du contenu de la mémoire supplémentaire (29) ou
c) avec les deux en même temps.

7. Appareil pour produire des images ultrasonores selon la revendication 6, caractérisé par le fait que dans les liaisons allant de l'étage mélangeur (31) à l'applicateur (1), d'une part, et à la sortie de lecture de la mémoire supplémentaire (29), d'autre part, sont prévues respectivement des mémoires-tampons (32, 34).

8. Appareil pour produire des images ultrasonores selon la revendication 6 ou 7, caractérisé par le fait que la commande des commutateurs de sélection (33, 35) prévue en avant de l'étage mélangeur (31), est opérée à la main, par exemple sous la forme de commutateurs à touches, ou automatiquement, par exemple en fonction de signaux de commande du circuit de commande (30) pour la mémoire supplémentaire (29).

Fig.1

Fig.2